# EUROPÄISCHE PATENTSCHRIFT

(11) **EP 1 015 408 B1**
(45) Veröffentlichungstag und Bekanntmachung des Hinweises auf die Patenterteilung: **27.03.2002**
(21) Anmeldenummer: 98945292.5
(22) Anmeldetag: 27.08.1998
(51) Int. Cl.: C07C 51/377, C07C 61/20, C07C 67/333, C07C 69/74, C07C 45/54, C07C 49/395

(54) **VERFAHREN ZUR HERSTELLUNG VON CYCLOPENTANON UND CYCLOPENTEN-1-CARBONSÄURE UND DEREN ESTERN**
METHOD FOR PRODUCING CYCLOPENTANONE AND CYCLOPENTENE-1-CARBOXYLIC ACID AND THEIR ESTERS
PROCEDE DE PRODUCTION DE CYCLOPENTANONE ET D'ACIDE CYCLOPENTENE-1-CARBOXYLIQUE ET DE LEURS ESTERS

(30) Priorität: 09.09.1997 DE 19739441
(43) Veröffentlichungstag der Anmeldung: 05.07.2000
(73) Patentinhaber: BASF AKTIENGESELLSCHAFT, 67056 Ludwigshafen (DE)
(72) Erfinder: FISCHER, Rolf, Hartmuth, D-69121 Heidelberg (DE); LIANG, Shelue, D-67071 Ludwigshafen (DE); PINKOS, Rolf, D-67098 Bad Dürkheim (DE); STEIN, Frank, D-67098 Bad Dürkheim (DE)
(86) Internationale Anmeldenummer: EP9805442
(87) Internationale Veröffentlichungsnummer: WO9912883

(56) Entgegenhaltungen:
- CHEMICAL ABSTRACTS, vol. 79, no. 5, 6. August 1973 Columbus, Ohio, US; abstract no. 31536q, ODINOKOV V.N. ET AL.: "Ozonolysis of alkenes and study of reactions of polyfunctional compounds.II. Preparation of 5-formynvaleric acid." Seite 414; Spalte l; XP002091058 & ODINOKOV V.N. ET AL.: ZH.ORG.KHIM., Bd. 9, Nr. 4, 1973, Seiten 671-673,

## Beschreibung

Die Erfindung betrifft ein Verfahren zur Herstellung von Cyclopentanon und Cyclopenten-1-carbonsäure und ihren Estern durch Umsetzung von 5-Formylvaleriansäure und ihren Estern und/oder 6-Hydroxycapronsäure und ihren Estern und/oder Verbindungen, die mit Wasser oder Alkoholen unter den Reaktionsbedingungen in 6-Hydroxycapronsäure bzw. ihre Ester übergehen können, allein oder im Gemisch mit Adipinsäureestern an oxidischen Katalysatoren bei Temperaturen von 200 bis 450°C in der Gas- oder Flüssigphase.

Aus EP-A-251 111 ist es bekannt, Cyclopentanon durch Umsetzung in der Gas- oder Flüssigphase von Adipinsäurediestern an oxidischen Katalysatoren bei erhöhter Temperatur herzustellen. Ferner ist aus EP-A-266 687 bekannt, diese Reaktion mit zeolithischen Katalysatoren oder mit Phosphatkatalysatoren durchzuführen.

Obgleich diese Verfahren technisch sehr vorteilhaft sind und Adipinsäurediester durch Veresterung von Adipinsäure gut zugänglich sind, bestand die Aufgabe, Cyclopentanon aus noch leichter zugänglichen Ausgangsmaterialien auch unter Inkaufnahme einer Koppelproduktion eines weiteren Wertproduktes herzustellen.

Dieses Wertprodukt ist Cyclopenten-1-carbonsäure oder deren Ester, die bisher ziemlich umständlich durch Reduktion von Cyclopentanon-2-carbonsäureestern zu Cyclopentanol-2-carbonsäurestern und Wasserabspaltung hergestellt wurden (Heterocycles (1996), 47(1), Seiten 423-425.

Ein weiteres Verfahren zur Herstellung von Cyclopenten-1-carbonsäure durch Umsetzung von Formylvaleriansäure in Gegenwart von Pyridin und Acetanhydrid ist aus Chemical Abstracts, vol. 79, no. 5, 6. August 1973 Columbus Ohio US, abstract No. 31536q, Odinokov V.N. et al, bekannt.

Diese Aufgabe wurde erfindungsgemäß gelöst mit einem Verfahren zur Herstellung von Cyclopentanon und Cyclopenten-1-carbonsäure oder deren Estern der Formel I 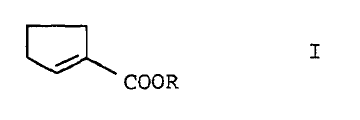 in der R Wasserstoff oder einen aliphatischen Rest mit 1 bis 6 C-Atomen oder einen cycloaliphatischen, araliphatischen oder aromatischen Rest mit 6 bis 12 C-Atomen bedeutet, gemäß Verbindungen der Formel II

X-(CH₂)₄-COOR II

in der X einen Formyl- oder Hydroxymethyl-Rest bedeutet und R die oben genannte Bedeutung hat und/oder Verbindungen, die mit Wasser oder Alkoholen ROH unter den Reaktionsbedingungen in Verbindungen der Formel II übergehen können, in der Gas- oder Flüssigpbase in Gegenwart von heterogenen oxidischen Katalysatoren auf Temperaturen von 200 bis 450°C erhitzt.

Nach einer besonderen Ausgestaltung des Verfahrens setzt man Gemische aus Verbindungen der Formel II mit Adipinsäurediestern der Formel III

ROCO-(CH₂)₄-COOR III III

um,
in der R die oben genannte Bedeutung hat, insbesondere Gemische wie sie gemäß dem Verfahren der DE-A 19 607 954 erhalten werden.

Die erfindungsgemäße Reaktion läßt sich z.B. für die Umsetzung von 5-Formylvaleriansäuremethylester zur Cyclopentanon und Cyclopenten-1-carbonsäuremethylester durch die folgende Reaktionsgleichung darstellen.

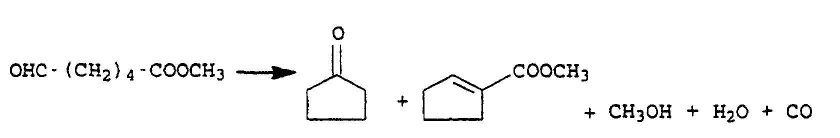

Bei Verwendung von 6-Hydroxycarbonsäure oder deren Estern oder von Verbindungen, die unter den Reaktionsbedingungen in diese übergehen, z.B. ε-Caprolacton, bedarf es zusätzlich einer gleichzeitigen katalytischen Dehydrierung.

In allen Fällen war es überraschend, daß diese Reaktion mit hohen Ausbeuten, Selektivitäten und Raum-Zeit-Ausbeuten abläuft.

Die als Ausgangsstoffe eingesetzten Verbindungen der Formel II sind 5-Formylvaleriansäure und 6-Hydroxycapronsäure und ihre Ester, allein oder im Gemisch mit Adipinsäurediester, wobei die Ester aliphatischen Reste mit 1 bis 6 Kohlenstoffatomen oder cycloaliphatische, aromatische Reste oder araliphatische Reste mit 5 bis 12, vorzugsweise 6 bis 8 C-Atomen, enthalten können. Beispiele für die Reste R sind Methyl-, Ethyl-, Propyl-, Isopropyl-, Butyl-, Isobutyl-, tert. Butyl-, Hexyl-, Cyclopentyl-, Cyclohexyl-, Phenyl- oder Benzylreste.

Beispielsweise kommen folgende Verbindungen der Formel II als Ausgangsstoffe in Betracht: 5-Formylvaleriansäure, 6-Hydroxycapronsäure, 5-Formylvaleriansäuremethylester, 5-Formylvaleriansäureethylester, 5-Formylvaleriansäure-i-butylester, 5-Formylvaleriansäurecyclohexylester, 5-Formylvaleriansäurebenzylester, 5-Formylvaleriansäurephenylester, 6-Hydroxycapronsäure, 6-Hydroxycapronsäuremethylester, 6-Hydroxycapronsäurepropylester, 6-Hydroxycapronsäure-n-butylester, 6-Hydroxycapronsäurecyclopentylester, 6-Hydroxycapronsäurephenylester allein oder im Gemisch mit Adipinsäuredimethylester, Adipinsäurediethylester oder Adipinsäuredi-n-butylester.

Als Ausgangsverbindungen kommen auch Gemische aus Verbindungen der Formel II mit Formyl- und Hydroxymethylgruppen in Betracht.

Mögliche Edukte sind auch Verbindungen, die unter den Reaktionsbedingungen Verbindungen der Formel II bilden. So kann man anstelle von 6-Hydroxycapronsäure oder 6-Hydroxycapronsäureestern Gemische aus Caprolacton und Wasser oder Alkoholen verwenden. Fällt also, z.B. bei der erfindungsgemäßen Umsetzung von 6-Hydroxycapronsäurestern Caprolacton als Nebenprodukt an, so kann dieses nach Abtrennung zurückgeführt werden.

Die als Ausgangsverbindung zu verwendende 5-Formylvaleriansäure ist durch Hydroformylierung von 3- und 4-Pentensäure z.B. nach den Angaben von WO 97/08127 zugänglich. 5-Formylvaleriansäureester lassen sich durch Hydroformylierung von 3- und 4-Pentensäurestern, z.B. nach den Angaben von EP-A 556 681, herstellen.

6-Hydroxycapronsäure und 6-Hydroxycapronsäureester entstehen z.B. durch Spaltung von Caprolacton mit Wasser bzw. Alkoholen.

Nach einer besonderen Ausführungsform werden Gemische aus 6-Hydroxycapronsäureester und Adipinsäurediester verwendet, wie sie z.B. nach den in DE-A 19 607 954 beschriebenen Verfahren erhalten werden, wobei neben 6-Hydroxycapronsäureester und Adipinsäurediester noch weitere Verbindungen wie Caprolacton, 6-Alkoxycapronsäureester, Glutarsäurediester, 5-Hydroxyvaleriansäureester, 2-Oxocapronsäureester, 1,2-Cyclohexandiole, Valerolacton, ungesättigte Adipinsäurediester wie z.B. Dihydromuconsäurediester, 3-Hydroxypentansäureester, 4-Oxopentansäureester und 5-Oxohexansäureester vorliegen können. Diese Verbindungen beeinträchtigen im allgemeinen weder die erfindungsgemäße Reaktion, noch führen sie überraschenderweise nach destillativer Aufreinigung zu Einbußen der Produktqualität.

In der Regel liegt dabei der Anteil des Adipinsäurediesters an dem umzusetzenden Gemisch bei bis zu 95, vorzugsweise bis zu 90 Gew.-%.

Als Katalysatoren sind saure oder basische Katalysatoren, aber auch solche, die sowohl saure als auch basische Eigenschaften besitzen, geeignet. Bei Verwendung von 6-Hydroxycapronsäure und ihren Estern als Ausgangsverbindungen müssen die Katalysatoren zusätzlich dehydrierende Eigenschaften besitzen.

Unter oxidischen Katalysatoren werden nicht nur Oxide im engeren Sinne verstanden sondern auch komplexe Sauerstoff enthaltende Verbindungen, die von sich aus saure oder basische Eigenschaften aufweisen oder entsprechend dotiert werden können. Dementsprechend kommen auch Heteropolysäuren, z.B. auf einem Träger aufgebracht, Zeolithe, die zur sauren Wirkung in der H-Form vorliegen und zur alkalischen Wirkung mit Alkali dotiert sind, Metallphosphate oder Verbindungen wie Carbonate der Hydroxide in Betracht, die in Oxide übergehen können.

Als oxidische Katalysatoren kommen z.B. Oxide von Elementen der 1. bis 14. Gruppe des periodischen Systems der Elemente oder Oxide der Seltenen Erdmetalle oder Gemische der genannten Oxide in Betracht. So sind beispielsweise Alkalioxide wie Natriumoxid, Erdalkalioxide, wie Magnesiumoxid, Calciumoxid, Bariumoxid, weiterhin Bortrioxid, Aluminiumoxid, Siliciumdioxid, z.B. in Form von Kieselgel, Kieselgut, Silikaten oder Quarz, ferner Zinndioxid, Bismutoxid, Kupferoxid, Zinkoxid, Lanthanoxid, Titandioxid, Zirkondioxid, Vanadiumoxide, Chromoxide, Molybdänoxide, Wolframoxide, Manganoxide, Eisenoxide, Ceroxide, Neodymoxide oder Gemische derartiger Oxide geeignet. Die Katalysatoren können noch durch Aufbringen von Zusätzen, wie Säuren (z.B. Phosphorsäure) oder Basen (z.B. Natriumhydroxid) modifiziert werden.

Im einzelnen sind z.B. La₂O₃, ZrO₂, Cr₂O₃/ZrO₂, CaO/ZnO, MgO/ZnO, K₂O/TiO₂, La₂O₃/Al₂O₃ und ZrO₂-SO₄ zu nennen.

Die erfindungsgemäß zu verwendenden Heteropolysäuren enthalten als wesentliches Element Wolfram oder bevorzugt Molybdän, wobei die partielle Substitution durch Vanadium möglich ist. Bei Verwendung von Vanadium sind die bevorzugten Atomverhältnisse V : Mo 1 : 6 bis 1 : 12. Beispiele für die Zentralatome sind Phosphor, Silizium, Arsen, Germanium, Bor, Titan, Cer, Thorium, Mangan, Nickel, Tellur, Iod, Cobalt, Chrom, Eisen, Gallium, Vanadium, Platin, Beryllium und Zink. Bevorzugt ist Phosphor und Silizium. Bevorzugtes Verhältnis von Molybdän- bzw. Wolframatomen zum jeweiligen Zentralatom ist 2,5 : 1 bis 12 : 1, bevorzugt 11 : 1 bis 12 : 1.

Als Molybdän-haltige Heteropolysäuren seien folgende Verbindungen im einzelnen beispielhaft genannt:
Dodecamolybdatophosphorsäure (H₃PMO₁₂O₄₀ *n H₂O), Dodecamolybdatokieselsäure (H₄SiMo₁₂O₄₀ *n H₂O), Dodecamolybdatocer(IV)säure (H₈CeMo₁₂O₄₂ *N H₂O), Dodecamolybdatoarsen(V)säure (H₃AsMo₁₂O₄₂ *N H₂O), Hexamolybdatochrom(III)säure (H₃CrMo₆O₂₄H₆ *N H₂O), Hexamolybdatonickel(II)säure (H₄NiMo₆O₂₄H₆ *5 H₂O), Hexamolybdatojodsäure (H₅JMo₆O₂₄ *n H₂O), Octadecamolybdatodiphosphorsäure (H₆P₂Mo₁₈O₆₂ *11 H₂O), Octadecamolybdatodiarsen(V)säure (H₆As₂Mo₁₈O₆₂ *25 H₂O), Nonamolybdatomangan(IV)säure (H₆MnMo₉O₃₂ *n H₂O), Undecamolybdatovanadatophosphorsäure (H₄PMo₁₁VO₄₀ *n H₂O), Decamolybdatodivanadatophosphorsäure (H₅PMo₁₀V₂O₄₀ *n H₂O), Hexamolybdatohexawolframatophosphorsäure (H₃PMo₆W₆O₄₀ *n H₂O).

Selbstverständlich können auch Mischungen von Heteropolysäuren eingesetzt werden. Bevorzugt werden Dodecamolybdatophosphorsäure und Dodecamolybdatokieselsäure verwendet.

Neben den freien Heteropolysäuren ist es aber auch möglich, deren Salze, insbesondere deren Alkalimetall- und Erdalkalimetallsalze als Katalysatoren zu benutzen. Bevorzugt sind die Cäsiumsalze. Wie bei den freien Säuren können auch bei Salzen entsprechende Mischungen verwendet werden.

Die Heteropolysäuren und deren Salze sind bekannte Verbindungen und können nach bekannten Verfahren, beispielsweise nach den Methoden von Brauer (Herausgeber): Handbuch der Präparativen Anorganischen Chemie, Band III, Enke, Stuttgart, 1981 oder nach den Verfahren von Top. Curr. Chem. 76, 1 (1978), hergestellt werden. Besonders bevorzugt sind Präparationsmethoden, die ohne den Einsatz organischer Lösungsmittel auskommen und stattdessen in wäßriger Lösung durchgeführt werden.

Die so hergestellten Heteropolysäuren liegen im allgemeinen in hydratisierter Form vor und werden vor ihrer Verwendung vom darin enthaltenen koordinativ gebundenen Wasser befreit. Die Dehydratisierung kann vorteilhaft thermisch, beispielsweise nach dem Verfahren von Makromol. Chem. 190, 929 (1989) durchgeführt werden. Eine andere Möglichkeit zur Dehydratisierung der Heteropolysäuren besteht je nach verwendeter Heteropolysäure darin, daß man die Heteropolysäure in einem organischen Lösungsmittel, beispielsweise in einem Dialkylether oder Alkohol, löst, das Wasser mit dem organischen Lösungsmittel aus seiner koordinativen Bindung mit der Heteropolysäure verdrängt und azeotrop mit dem Lösungsmittel abdestilliert.

Nach diesen Methoden hergestellte, wasserfreie Heteropolysäuren werden in der Regel anschließend bei Temperaturen von 250 bis 500°C, bevorzugt 280 bis 400°C calciniert. Die Calcinierung erfolgt je nach Temperatur und gewähltem Druck in der Regel zwischen einer Stunde und 24 Stunden. Die so erhaltenen Katalysatoren können direkt in dem erfindungsgemäßen Verfahren eingesetzt werden.

Die Heteropolysäure-Katalysatoren werden bevorzugt auf Träger aufgebracht. Dazu wird die Heteropolysäure auf ein Trägermaterial, wie Aktivkohle, Siliciumdioxid, Titandioxid oder Zirkoniumdioxid, nach an sich bekannten Methoden aufgebracht, beispielsweise indem man das betreffende Trägermaterial mit einer Lösung der Heteropolysäure in einem Lösungsmittel, vorzugsweise Wasser, tränkt und anschließend bei Temperaturen von 100 bis 250°C, vorzugsweise 130 bis 250°C unter reduziertem Druck so lange trocknet, bis kein Wasser mehr im Katalysator nachweisbar ist. Nach diesen Methoden hergestellte, wasserfreie Heteropolysäuren werden anschließend bei Temperaturen von 250 bis 500°C, bevorzugt 280 bis 400°C calciniert.

Als Zeolithe kommen beliebige Zeolithe mit sauren oder basischen Zentren in Betracht.

Im Falle der basischen Eigenschaften aufweisenden Zeolithe verwendet man z.B. Alkali oder Erdalkali enthaltende Zeolithe, im Falle der sauren Eigenschaften aufweisende Zeolithe, solche der sauren H-Form, bei denen Alkaliionen durch Wasserstoffionen ausgetauscht sind.

Bevorzugt sind 12-Ring-Zeolithe vom Strukturtyp BETA, Y, EMT und Mordenit, sowie 10-Ring-Zeolithe vom Typ Pentasil. Die Zeolithe können neben den Elementen Aluminium und Silicium auch Bor, Gallium, Eisen oder Titan im Gerüstgitter enthalten. Sie können darüber hinaus auch mit Elementen der Gruppen 3 und 8 bis 13, sowie den Elementen der Lanthaniden partiell ausgetauscht sein.

Als Zeolithe kommen für die Verwendung als Katalysator, z.B. solche der Strukturtypen MFT, MEL, BOG, BEA, EMT; MOR, FAU, MTW, LTL, NES, CON oder MCM-22 entsprechend der Strukturklassifizierung aus W.M. Meier, D.H. Olson, Ch. Baerlocher, Atlas of zeolithe Structur Types, Elsevier, 4^{th} ed., 1996, in Betracht.

Im einzelnen kommen insbesondere die Zeolithe ZBM-20, Fe-H-ZSM5, Sn-Beta-Zeolith, Beta-Zeolith, Zr-Beta-Zeolith, H-Beta-Zeolith, H-Mordenit, USY, Ce-V-Zeolith, H-Y-Zeolith, Ti/B-Betazeolith, B-Beta-Zeolith oder ZB-10 in Betracht.

Um eine möglichst hohe Selektivität, hohe Umsätze sowie lange Standzeiten zu erreichen, ist es vorteilhaft, die Zeolithe zu modifizieren. Eine geeignete Modifizierung der Katalysatoren besteht z.B. darin, daß man den unverformten oder verformten Zeolithen mit Metallsalzen durch einen Inonenaustausch oder durch Imprägnierung dotiert. Als Metalle werden Alkalimetalle wie Li, Cs, K, Erdalkalimetalle wie Mg, Ca, Sr. Metalle der 3., 4. und 5. Hauptgruppe wie Al, Ga, Ge, Sn, Pb, Bi, Übergangsmetalle der 4. - 8. Nebengruppe wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe wie Cu, Ag, Zn, Seltene Edelmetalle wie La, Ce, Pr, Nd, Er, Yb und U eingesetzt.

Zweckmäßigerweise führt man die Dotierung so durch, daß man den verformten Zeolithen in einem Steigrohr vorlegt und bei 20 bis 100°C eine wäßrige oder ammoniakalische Lösung eines Halogenids oder eines Nitrats der voranbeschriebenen Metalle überleitet. Ein derartiger Ionenaustausch kann an der Wasserstoff-, Ammonium- und Alkaliform des Zeolithen vorgenommen werden. Eine weitere Möglichkeit der Metallaufbringung auf den Zeolithen ist gegeben, indem man das zeolithische Material, z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man Cu(NO₃)₂ x 3 H₂O oder Ni(NO₃)₂ x 6 H₂O oder Ce(NO₃)₃ x 6 H₂O oder La(NO₃)₂ x 6 H₂O oder Cs₂CO₃ in Wasser löst und mit dieser Lösung den verformen oder unverformten Zeolith eine gewisse Zeit, z.B. 30 Minuten, tränkt. Die eventuell überstehende Lösung wird im Rotationsverdampfer von Wasser befreit. Danach wird der getränkte Zeolith bei etwa 150°C getrocknet und bei etwa 550°C calciniert. Dieser Tränkvorgang kann mehrmals hintereinander vorgenommen werden, um den gewünschten Metallgehalt einzustellen.

Es ist auch möglich, eine wäßrige Ni(NO₃)₂-Lösung oder ammoniakalische Pd(NO₃)₂-Lösung herzustellen und darin den reinen pulverförmigen Zeolithen bei 40 bis 100°C unter Rühren etwa 24 h aufzuschlämmen. Nach Abfiltrieren, Trocknen bei etwa 150°C und Calcinierung bei etwa 500°C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber z.B. eine wäßrige Ni(NO₃)₂-Lösung oder ammoniakalische Pd(NO₃)₂-Lösung bei leicht erhöhter Temperatur zwischen 30 und 80°C im Kreislauf 15 bis 20 h leitet. Danach wird mit Wasser ausgewaschen, bei etwa 150°C getrocknet und bei etwa 550°C calciniert. Bei manchen metalldotierten Zeolithen, z.B. Pd-, Cu-, Ni- dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft, z.B. so vor, daß man Zeolithe in Pulverform mit 1 n Phosphorsäure 1 Stunde bei 80°C behandelt. Nach der Behandlung wird mit Wasser gewaschen, bei 110°C/16 Stunden getrocknet und bei 500°C/20 Stunden calciniert. Nach einer anderen Arbeitsweise behandelt man Zeolithe vor oder nach ihrer Verformung mit Bindemittel, z.B. 1 bis 3 Stunden bei Temperaturen von 60 bis 80°C mit einer 3 bis 25 gew.-%igen, insbesondere 12 bis 20 gew.-%igen wäßrigen Salzsäure. Anschließend wird der so behandelte Zeolith mit Wasser gewaschen, getrocknet und bei 400°C bis 500°C calciniert.

Weitere Katalysatoren für die Herstellung von Cyclopentanon sind Phosphate, insbesondere Aluminiumphosphate, Siliciumaliuminiumphosphate, Eisenaluminiumphosphate, Cerphospat, Zirkonphosphate, Borphosphat, Eisenphosphat, Calciumphosphat oder deren Gemische.

Als Aluminiumphosphat-Katalysatoren werden für das erfindungsgemäße Verfahren insbesondere unter hydrothermalen Bedingungen synthetisierte Aluminiumphosphate eingesetzt. Geeignete Aluminiumphosphate sind z.B. APO-5, APO-9, APO-11, APO-12, APO-14, APO-21, APO-25, APO-31 und APO-33.

Beispielsweise AlPO₄-5(APO-5) wird synthetisiert, indem man Orthophosphorsäure mit Pseudoboehmit (Catapal SB® ) in Wasser homogen mischt, zu dieser Mischung Tetrapropylammoniumhydroxid gibt und danach bei etwa 150°C 20 bis 60 h unter autogenem Druck in einem Autoklaven umsetzt. Das abfiltrierte AlPO₄ wird getrocknet bei 100 bis 160°C und calciniert bei 450 bis 550°C.

AlPO₄-9 (APO-9) wird ebenfalls aus Orthophosphorsäure und Pseudoboehmit aber in wäßriger DABCO-Lösung (1,4-Diazabicyclo-(2,2,2)-octan) bei ca. 200°C unter autogenem Druck während 200 bis 400 h synthetisiert. Verwendet man anstelle von DABCO-Lösung Ethylendiamin, so gelangt man zu APO-12.

Die Synthese des AlPO₄-21 (APO-21) erfolgt aus Orthophosphorsäure und Pseudoboehmit in wäßriger Pyrrolidin-Lösung bei 150 bis 200°C unter autogenem Druck während 50 bis 200 h.

Für das erfindungsgemäße Verfahren kann man auch bekannte Siliciumaluminiumphosphate wie SAPO-, SAPO-11, SAPO-31 und SAPO-34 einsetzen. Diese Verbindungen werden hergestellt durch Kristallisation aus wäßriger Mischung bei 100 bis 250°C und autogenem Druck während 2 h bis 2 Wochen, wobei die Reaktionsmischung aus einer Silicium-, Aluminium- und Phosphorkomponente in wäßrigen aminhaltigen Lösungen umgesetzt wird.

SAPO-5 beispielsweise wird durch Mischen von SiO₂, suspendiert in wäßriger Tetrapropylammoniumhydroxid-Lösung, mit einer wäßrigen Suspension aus Pseudoboehmit und Orthophosphonsäure und anschließende Umsetzung bei 150 bis 200°C während 20 bis 200 h unter autogenem Druck in einem Rührautoklaven erhalten. Die Trocknung des abfiltrierten Pulvers erfolgt bei 110 bis 160°C und die Calcination bei 450 bis 550°C.

Als Silciumaluminiumphosphate sind auch ZYT-5, ZYT-6, ZYT-7, ZYT-9, ZYT-11, ZYT-12 geeignet. Als Phosphat-Katalysatoren kann man bei dem Verfahren gefällte Aluminiumphosphate einsetzen.

Beispielsweise wird ein derartiges Aluminiumphosphat hergestellt, indem 92 g Diammoniumhydrogenphosphat in 700 ml Wasser gelöst werden. Zu dieser Lösung wird 260 g Al(NO₃)₃ x 9 H₂O in 700 ml Wasser über 2 h zugetropft. Dabei wird der pH-Wert durch gleichzeitige Zugabe von 25 %iger NH₃-Lösung bei pH 8 gehalten. Der entstandene Niederschlag wird 12 h nachgerührt, dann abgesaugt und ausgewaschen. Er wird bei 60°C/16 h getrocknet.

Borphosphate als Katalysatoren für das erfindungsgemäße Verfahren kann man beispielsweise durch Mischen und Kneten von konzentrierter Borsäure und Phosphorsäure und durch anschließende Trocknung und Calcination in Inertgas-, Luft- oder Dampfatmosphäre bei 250 bis 650°C vorzugsweise 300 bis 500°C herstellen.

CePO₄ erhält man durch Fällung aus 52 g Ce(NO₃)₃ x 6 H₂O und 56 g NaH₂PO₄ x 2 H₂O. Nach der Filtration wird das Material zu Strängen verformt, bei 120°C getrocknet und bei 450°C calciniert. Als Phosphate lassen sich aus SrHPO₄, FePO₄ und Zr₃(PO₄)₄ für das erfindungsgemäße Verfahren verwenden.

Die hier beschriebenen Katalysatoren können z.B. als 2- bis 4- mm-Stränge oder als Tabletten mit z.B. 3 bis 5 mm Durchmesser oder als Splitt mit Teilchengrößen von z.B. 0,1 bis 0,5 mm oder als Wirbelkontakt eingesetzt werden.

Um bei Verwendung von Hydroxycapronsäureester die Katalysatoren mit Dehydrier-Aktivität zu versehen, verwendet man in der Regel zusätzlich Metalle wie Kupfer oder Silber auf oxidischen Trägern, wie Metalloxiden.

Es hat sich gezeigt, daß bei Verwendung von basischen Katalysatoren verstärkt Cyclopentanon, bei Verwendung von Katalysatoren mit sauren Eigenschaften verstärkt Cyclopenten-1-carbonsäureester I gebildet werden.

Die erfindungsgemäße Umsetzung kann ohne Zusatz von Wasser durchgeführt werden. Durch die Zugabe von Wasser wird eine Erhöhung von Selektivität und Standzeit erreicht. Das Molverhältnis von Ausgangsverbindung II zu Wasser beträgt hierbei vorteilhaft 1 : 0 bis 1 : 20, insbesondere 1 : 0,1 bis 1 : 5.

Die Umsetzung kann in der Gasphase oder in der flüssigen Phase, gegebenenfalls auch unter Mitverwendung von Verdünnungsmitteln durchgeführt werden. Als Verdünnungsmittel sind z.B. Lösungsmittel geeignet, die unter den Reaktionsbedingungen vollständig oder weitgehend inert sind, z.B. Ether wie Dioxan oder Tetrahydrofuran, Alkohole wie Methanol und Ethanol. Vorzugsweise wird in der Gasphase gearbeitet, sofern gut verdampfbare Ausgangsstoffe vorliegen.

Die Umsetzung kann diskontinuierlich oder kontinuierlich als Festbettreaktion mit Festbettkatalysatoren, beispielsweise in Sumpf- oder Rieselfahrweise in der Flüssig- oder Gasphase oder als Wirbelbettreaktion mit in auf- und abwirbelnder Bewegung befindlichen Katalysatoren in der Gasphase oder aber mit in der Flüssigphase suspendierten Katalysatoren durchgeführt werden.

Die Umsetzung findet bei Temperaturen von 200 bis 450, vorzugsweise bei 250 bis 390°C, insbesondere 300 bis 380°C statt. Im allgemeinen wird die Reaktion unter Atomosphärendruck durchgeführt. Es ist jedoch auch möglich, schwach verminderten oder schwach erhöhten Druck, z.B. bis zu 20 bar anzuwenden. Die Katalysatorbelastung liegt im allgemeinen bei 0,01 bis 40, vorzugsweise 0,1 bis 20 g Verbindung der Formel II je Gramm Katalysator und Stunde.

Die Umsetzung in der Flüssigphase wird beispielsweise so durchgeführt, daß man ein Gemisch aus der Ausgangsverbindung II und gegebenenfalls Wasser in Gegenwart eines suspendierten Festbettkatalysators auf die gewünschte Reaktionstemperatur erhitzt. Nach Ablauf der notwendigen Reaktionszeit wird das Reaktionsgemisch abgekühlt und der Katalysator, z.B. durch Filtration, entfernt. Das Reaktionsgemisch wird anschließend zur Gewinnung der Wertprodukte bzw. des unumgesetzten Esters fraktionierend destilliert. Es ist weiterhin möglich, die im Laufe der Umsetzung entstehenden Reaktionsprodukte durch Destillation kontinuierlich aus dem Reaktionsgemisch abzutrennen.

Gemäß einer bevorzugten Ausführungsform des erfindungsgemäßen Verfahrens in der Gasphase verdampft man zunächst ein Gemisch aus der Ausgangsverbindung II und gegebenenfalls Wasser und leitet es dann, gegebenenfalls zusammen mit Wasserstoff oder einem Inertgas, wie Stickstoff, Kohlendioxid oder Argon, bei der gewünschten Reaktionstemperatur gasförmig in eine in auf- und abwirbelnder Bewegung befindliche Katalysatorschicht ein.

Eine weitere bevorzugte Ausführungsform in der Gasphase besteht z.B. darin, daß man ein Gemisch aus der Ausgangsverbindung II und gegebenenfalls Wasser zunächst verdampft und es dann, gegebenenfalls zusammen mit einem Inertgas wie z.B. Stickstoff, Kohlendioxid oder Argon bei der gewünschten Reaktionstemperatur gasförmig in Sumpf- oder Rieselfahrweise über eine festangeordnete Katalysatorschicht leitet.

Der Reaktionsaustrag wird mittels geeigneter Kühlvorrichtungen kondensiert und anschließend durch fraktionierende Destillation aufgearbeitet. Unumgesetzte Ausgangsverbindungen können zurückgeführt werden.

Das nach dem erfindungsgemäßen Verfahren erhältliche Cyclopentanon ist ein wertvolles Zwischenprodukt. So läßt sich z.B. aus Cyclopentanon durch reduktive Aminierung Cyclopentylamin herstellen, das für die Synthese von Pflanzenschutzmitteln und Pharmazeutika von Interesse ist.

Cyclopenten-1-carbonsäureester sind wertvolle Bausteine für die Synthese von Zwischenprodukten.

### Beispiele

Die Prozentangaben zur Charakterisierung der Katalysatoren bedeuten Gewichtsprozente.
a) Herstellung der Katalysatoren:
   - ZrO₂:: Firma Norton (SN 9516321); als solches direkt eingesetzt.
   - La₂O₃(3 % La)/ZrO₂: ZrO₂ (Norton, SN 9516321) wurde mit La(NO₃)₃-Lösung getränkt, 4 Stunden bei 120°C getrocknet und 6 Stunden bei 400°C calciniert.
   - La₂O₃(10 % La)/α·Al₂O₃: : α-Al₂O₃ (Norton) wurde mit La(NO₃)₃-Lösung getränkt, 4 Stunden bei 120°C getrocknet und 6 Stunden bei 400°C calciniert.
   - 95 % ZnO/5 % MgO: Fa BASF (H5-10)
   - 56 % ZnO/44 % CaO: Fa BASF (H5-11)
   - Al₂O₃: Fa BASF (D 10-10)
b) Versuchsdurchführung für die Beispiele in Tab. 1;
   In einem Gasphasenreaktor wurden 100 ml Katalysator mit 30 ml Quarzringen als Verdampferstrecke überschichtet. Bei den angegebenen Temperaturen wurden pro Stunde 10 g Ausgangsstoff, entsprechende Mengen Wasser (Tab. 1) oder Methanol bzw. 50 NL Stickstoff (Beispiele 18-22) oder Wasserstoff (Beispiele 1-17) von oben nach unten über den Katalysator geleitet. Die Reaktionsausträge wurden in einer Vorlage unter Kühlung mit Trockeneis/Aceton kondensiert. Die Zusammensetzung der Reaktionsausträge und somit die Umsätze sowie die Selektivitäten wurden gaschromatographisch ermittelt (Tabelle 1).

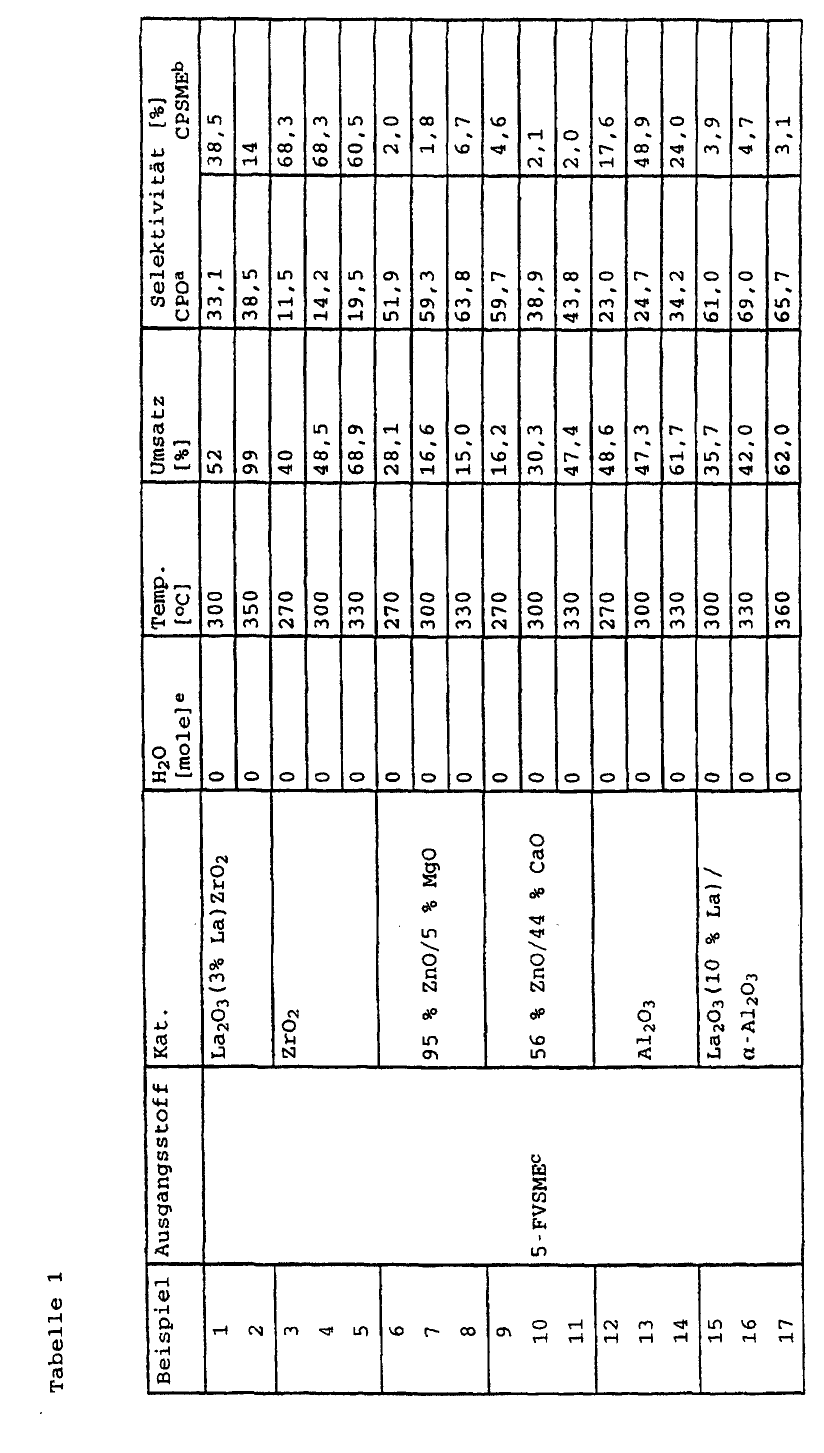

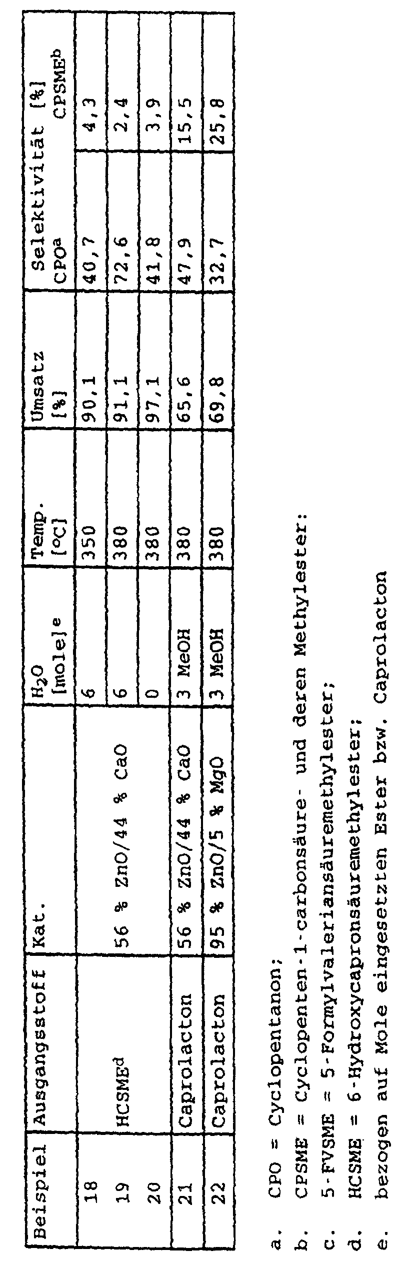

### Versuchsdurchführung für die Beispiele in Tabelle 2:

Die in Tab. 2 aufgeführten Edukte stammen aus Stufe 12 des in DE-A 19 607 954 beschriebenen Verfahrens. In einem Gasphasenreaktor wurden 100 ml (BASF; H 5-11) eines Katalysators der Zusammensetzung 56 Gew.-% ZnO, 44 Gew.-% CaO - mit 30 ml Quarzringen als Verdampferstrecke überschichtet. Bei 380 - 400°C wurden pro Stunde 10 g der in der Tabelle 2 angegebenen Edukte und 20 NL Stickstoff von oben nach unten über den Katalysator geleitet. Die Reaktionsausträge wurden in einer Vorlage unter Kühlung mit Trokkeneis/Aceton kondensiert. Der Reaktionsaustrag wurde während eines Zeitraumes von 7 Stunden gesammelt. Die Angaben bzgl. Cyclopentanon-Selektivität beziehen sich auf umgesetzten Adipinsäuredimethylester, 6-Hydroxycapronsäuremethylester und gegebenenfalls auf Caprolacton.

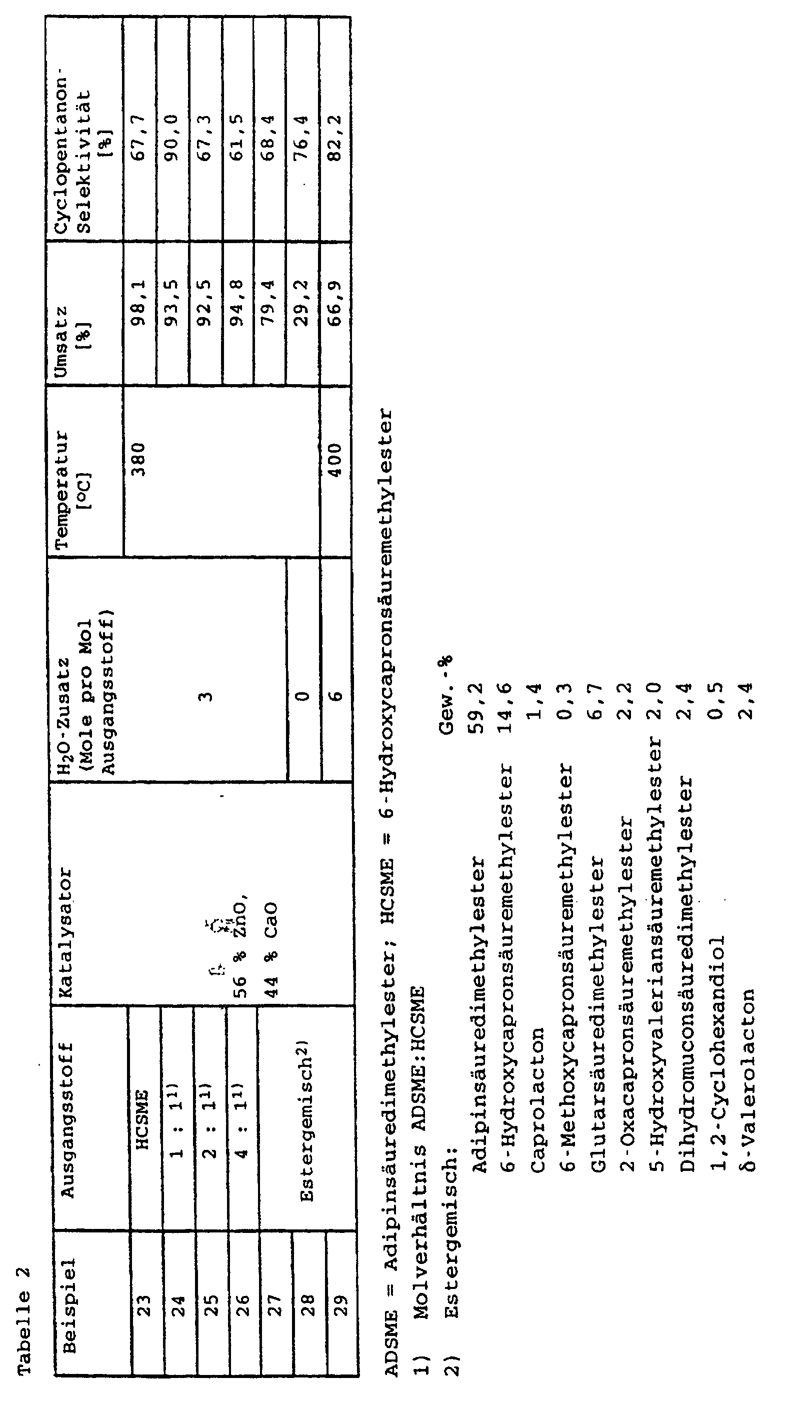

## Patentansprüche

1. Verfahren zur Herstellung von Cyclopentanon und Cyclopenten-1-carbonsäure oder deren Estern der Formel I 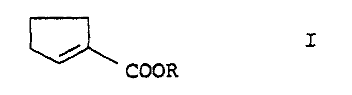 in der R Wasserstoff oder einen aliphatischen Rest mit 1 bis 6 C-Atomen oder einen cycloaliphatischen, araliphatischen oder aromatischen Rest mit 6 bis 12 C-Atomen bedeutet, **dadurch gekennzeichnet, daß** man Verbindungen der Formel II
X-(CH₂)₄-COOR II
in der X einen Formyl- oder Hydroxymethyl-Rest bedeutet und R die oben genannte Bedeutung hat und/oder Verbindungen, die mit Wasser oder Alkoholen ROH unter den Reaktionsbedingungen in Verbindungen der Formel II übergehen können, in der Gas- oder Flüssigphase in Gegenwart von heterogenen oxidischen Katalysatoren auf Temperaturen von 200 bis 450°C erhitzt.

2. Verfahren gemäß Anspruch 1, **dadurch gekennzeichnet, daß** man die Umsetzung der Verbindungen der Formel II im Gemisch mit Adipinsäurediestern der Formel III
ROCO-(CH₂)₄-COOR III,
in der R die oben genannte Bedeutungen hat, durchführt.

3. Verfahren gemäß Anspruch 1, **dadurch gekennzeichnet, daß** man als Verbindungen, die unter den Reaktionsbedingungen mit Wasser oder Alkoholen in die Verbindungen der Formel II übergehen, ε-Caprolacton verwendet.

4. Verfahren gemäß Anspruch 1, **dadurch gekennzeichnet, daß** man als Edukt ein Gemisch aus 6-Hydroxycapronsäureester und Adipinsäurediester verwendet.

5. Verfahren gemäß Anspruch 1, **dadurch gekennzeichnet, daß** man die Umsetzung unter Zusatz von Wasser durchführt.

6. Verfahren gemäß Anspruch 1, **dadurch gekennzeichnet, daß** man bei Verwendung von Hydoxycapronsäure oder deren Estern einen Katalysator mit zusätzlicher Dehydrieraktivität verwendet.

7. Verfahren gemäß Anspruch 1, **dadurch gekennzeichnet, daß** man zur Herstellung von vorwiegend Cyclopentanon basische oxidische Katalysatoren verwendet.

8. Verfahren gemäß Anspruch 1, **dadurch gekennzeichnet, daß** man zur Herstellung von vorwiegend Cyclopentencarbonsäure oder deren Estern saure oxidische Katalysatoren verwendet.

9. Verfahren gemäß Anspruch 1, **dadurch gekennzeichnet, daß** man als Katalysatoren Zeolithe verwendet.

10. Verfahren gemäß Anspruch 1, **dadurch gekennzeichnet, daß** man als Katalysatoren Heteropolysäuren verwendet.

11. Verfahren gemäß Anspruch 1, **dadurch gekennzeichnet, daß** man als Katalysatoren Phosphate verwendet.

12. Verfahren gemäß Anspruch 1, **dadurch gekennzeichnet, daß** man als Katalysatoren Metalloxide der 1. bis 14. Gruppe des Periodensystems der Elemente und/oder Oxide der Lanthaniden verwendet.

## Claims

1. A process for preparing cyclopentanone and cyclopentene-1-carboxylic acid or an ester thereof of the formula I 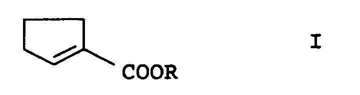 where R is hydrogen or an aliphatic radical having 1-6 carbon atoms or a cycloaliphatic, araliphatic or aromatic radical having 6-12 carbon atoms, which comprises heating a compound of the formula II
X-(CH₂)₄-COOR II
where X is formyl or hydroxymethyl and R is defined as above, and/or a compound which is converted into a compound of the formula II by reaction with water or alcohols ROH under the reaction conditions to from 200 to 450°C in the gas or liquid phase in the presence of a heterogeneous oxidic catalyst.

2. A process as claimed in claim 1, wherein a compound of the formula II is reacted in a mixture with an adipic diester of the formula III
ROCO-(CH₂)₄-COOR III,
where R is defined as above.

3. A process as claimed in claim 1, wherein the compound which is converted into a compound of the formula II is ε-caprolactone.

4. A process as claimed in claim 1, wherein the starting material used is a mixture of 6-hydroxycaproic ester and adipic diester.

5. A process as claimed in claim 1, wherein water is added to the reaction mixture.

6. A process as claimed in claim 1, wherein the catalyst used when using hydroxycaproic acid or a hydroxycaproic ester has additional dehydrogenation activity.

7. A process as claimed in claim 1, wherein a basic oxidic catalyst is used to give predominantly cyclopentanone.

8. A process as claimed in claim 1, wherein an acidic oxidic catalyst is used to give predominantly cyclopentene carboxylic acid or an ester thereof.

9. A process as claimed in claim 1, wherein the catalyst used is a zeolite.

10. A process as claimed in claim 1, wherein the catalyst used is a heteropolyacid.

11. A process as claimed in claim 1, wherein the catalyst used is a phosphate.

12. A process as claimed in claim 1, wherein the catalyst used is a metal oxide of groups 1-14 of the Periodic Table of the Elements and/or a lanthanide oxide.

## Revendications

1. Procédé de production de cyclopentanone et d'acide cyclopentène-1-carboxylique ou de ses esters de formule I 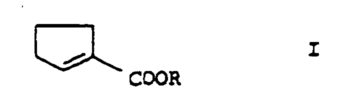 dans laquelle R représente un atome d'hydrogène ou un résidu aliphatique avec 1 à 6 atomes de carbone, ou un résidu cycloaliphatique, araliphatique ou aromatique avec 6 à 12 atomes de carbone, **caractérisé en ce que** l'on chauffe des composés de formule II,
X-(CH₂)₄-COOR II
dans laquelle X représente un résidu formyle ou hydroxyméthyle, R ayant la même signification que ci-dessus, et/ou des composés qui peuvent se convertir avec de l'eau ou des alcools ROH, dans les conditions de la réaction, en composés de formule II, en phase gazeuse ou liquide, en présence de catalyseurs d'oxydation hétérogènes, à des températures pouvant aller jusqu'à 200°C à 450°C.

2. Procédé selon la revendication 1, **caractérisé en ce que** l'on réalise la mise en réaction des composés de formule II mélangés à des diesters de l'acide adipique de formule III
ROCO-(CH₂)₄-COOR III,
dans laquelle R a la même signification que ci-dessus.

3. Procédé selon la revendication 1, **caractérisé en ce que** l'on met en oeuvre le ε-caprolactone en tant que composé se convertissant en composé de formule II avec l'eau ou les alcools, dans les conditions de la réaction.

4. Procédé selon la revendication 1, **caractérisé en ce que** l'on met en oeuvre en tant qu'éduit un mélange d'ester de l'acide 6-hydroxycaproïque et de diester de l'acide adipique.

5. Procédé selon la revendication 1, **caractérisé en ce que** l'on réalise la mise en réaction en ajoutant de l'eau.

6. Procédé selon la revendication 1, **caractérisé en ce que**, en cas d'utilisation de l'acide hydroxycaproïque ou des esters de ce dernier, l'on met en oeuvre un catalyseur doté d'une activité de déshydrogénation supplémentaire.

7. Procédé selon la revendication 1, **caractérisé en ce que**, pour la production de cyclopentanone principalement, l'on met en oeuvre des catalyseurs d'oxydation basiques.

8. Procédé selon la revendication 1, **caractérisé en ce que**, pour la production d'acide cyclopentènecarboxylique ou des esters de ce dernier principalement, l'on met en oeuvre des catalyseurs d'oxydation acides.

9. Procédé selon la revendication 1, **caractérisé en ce que** l'on met en oeuvre des zéolithes en tant que catalyseurs.

10. Procédé selon la revendication 1, **caractérisé en ce que** l'on met en oeuvre des hétéropolyacides en tant que catalyseurs.

11. Procédé selon la revendication 1, **caractérisé en ce que** l'on met en oeuvre des phosphates en tant que catalyseurs.

12. Procédé selon la revendication 1, **caractérisé en ce que** l'on met en oeuvre en tant que catalyseurs des oxydes métalliques du groupe 1 à 14 du Tableau Périodique des Eléments et/ou des oxydes des lanthanides.
